# EUROPEAN PATENT SPECIFICATION

(11) **EP 1 976 579 B1**
(45) Date of publication and mention of the grant of the patent: **07.08.2013**
(21) Application number: 06784511.5
(22) Date of filing: 31.05.2006
(51) Int. Cl.: A61M 1/14

(54) **DUAL PURPOSE ACUTE AND HOME TREATMENT DIALYSIS MACHINE**
DIALYSEGERÄT MIT DOPPELTER FUNKTION FÜR DIE AKUTBEHANDLUNG UND DIE BEHANDLUNG ZU HAUSE
APPAREIL DE DIALYSE DOUBLE USAGE POUR TRAITEMENT DE COURTE DUREE ET TRAITEMENT A DOMICILE

(30) Priority: 06.01.2006 US 757052 P
(43) Date of publication of application: 08.10.2008
(73) Proprietor: Renal Solutions, Inc., Warrendale PA 15086 (US)
(72) Inventor: DeCOMO, Peter, Cranberry Township, PA 16066 (US)
(74) Representative: Harguth, Alexander
(86) International application number: PCT/US2006/021056
(87) International publication number: WO 2007/081384

(56) References cited:
- EP-A- 1 576 972
- WO-A-97/47336
- US-A- 5 609 770
- US-A- 5 776 345
- US-A- 5 863 421

## Description

### BACKGROUND

The present invention relates to a dialysis machine for extracorporeal treatment of blood. More particularly, the invention relates to a dialysis machine that provides distinct modes of operation, e.g., a home setting or an acute setting (e.g., a hospital or dialysis clinic) e.g. see US 5863421.

Various types of medical equipment are designed with the knowledge that the equipment may be used in an ambulatory manner. For example, U.S. Patent No. 5,317,506 issued to Coutre et al. and entitled Infusion Fluid Management System discloses an infusion management and pumping system. Infusion prescriptions are generated and monitored by a pharmacy management system. Labels for each infusion to be given to a patient are generated and printed in a bar code format. Each label contains data regarding a prescribed infusion program, including the drug or drugs to be infused, the infusion regimen, the expiration date, and the patient to whom the infusion is to be administered. The management system checks for incompatibilities between drugs that are being prescribed for simultaneous infusion. Each label generated by the management system is attached to the container which holds the infusion solution. The data on the label is transferred to an infusion pumping system by a bar code reader at the infusion pumping system. The pumping system checks that all necessary data has been entered. During operation, the pumping system checks for a variety of alarm conditions and stores any alarms in a ranking according to urgency. The infusion pumping system is responsive to remote or biofeedback instructions to alter the planned infusion program. Central computer records processing receives infusion data and provides infusion, inventory, and use analysis.

Another example is U.S. Patent No. 5,376,070 issued to Purvis et al. and entitled Data Transfer System for an Infusion Pump. That patent discloses a data transfer system for communication with an infusion pump of the type used for programmable delivery of medication such as insulin to a patient. The data transfer system includes a communication station having a shaped pocket formed therein for seated reception of the infusion pump. Optical communication members including light emitting and detecting devices mounted on the pump and station are aligned for two-way data transmission when the pump is seated within the station pocket. The communication station can be used directly to monitor data received from the pump, and to transmit reprogrammed data to the pump, as desired. Alternately, the communication station can provide a data relay link to a remote site such as to a computer via a computer data cable, or a modem. See also U.S. Patent No. 5,360,710 issued to Tune et al. and entitled Ambulatory Infusion Pump.

U.S. Patent No. 6,699,230 issued to Jaafar et al. and entitled Apparatus and Method for Out-Of-Hospital Thrombolytic Therapy discloses an apparatus and method for emergency administration or self-administration of thrombolytic therapy in early stage of a heart attack. The apparatus includes a needle injector for making a venipuncture, a battery operated micro cooler for maintaining low temperature environment for vials with lyophilized thrombolytic and adjuvant drugs, a container with a diluent for reconstitution of the lyophilized drugs, a programmable infusion pump, and a microprocessor for controlling the process of infusion and recording the data. As the system is activated, the container becomes fluidly communicable with the infusion pump and vials with drugs in the cooler. Designed for autonomous execution of several schedules of infusion, it also can be controlled remotely by a qualified operator via an Internet interface.

Finally, U.S. Patent No. 5,250,027 issued to Lewis et al. and entitled Peristaltic Infusion Device With Backpack Sensor relates to a sensed member and sensing member for use on a medical fluid infusion device and support device wherein the fluid infusion device is usable in a conventional manner such as on an IV pole and in an ambulatory manner such as in a support device for use in an ambulatory backpack. The sensing member of the present invention detects when the fluid infusion device is to be used in an ambulatory manner to adjust the operation of the infusion control member to account for the difference in fluid flow rates caused by the different fluid pressures within the fluid delivery set when the fluid infusion device is used in a conventional manner and an ambulatory manner.

Dialysis machines for the extracorporeal treatment of a patient's blood are well known. Such devices withdraw blood from a patient, circulate the blood through a treatment unit, and then return the treated blood to the patient. Dialysis machines have a number of variable parameters that are individually adjusted prior to each dialysis treatment in accordance with the prescribing physician's instructions for a particular patient. Such parameters include, for example, blood flow rate, the duration of treatment, the type of dialyzer used, the dialyzer values, the type of dialysate, the type of infusate, and values related to treatment with heparin or other anti-coagulant.

Setting up a dialysis machine prior to administration of dialysis to a patient can be a complex process. Each of the parameters identified above must be pre-set. In addition, the machine itself must be prepared. The dialyzer must be installed. Sources of heparin and saline must be connected. Separate sets of tubing must be installed to carry both blood and dialysate. The tubing must be flushed and primed. The tubing must be checked for the presence of air bubbles, and any air bubbles found must be removed. The dialysate must be warmed to a pre-determined temperature. Only when all parameters have been set and set-up is complete can dialysis treatment of a patient commence. Because of the complexity of the dialysis machine, set-up of a dialysis machine typically is performed by a trained professional

A need exists for a dialysis machine that can be safely operated in a patient's home or similar environment. This need is fulfilled by the invention as claimed.

### SUMMARY OF THE INVENTION

The present disclosure is directed to a method of operating a dialysis machine comprising receiving patient specific data at an input device, storing the received patient specific data in a non-volatile memory device, and implementing a treatment according to one of an acute care mode or a home care mode. In the case of the acute care mode of treatment, the patient specific data is deleted after implementing the treatment. In the case of the home care mode of treatment, the patient specific data is retained after implementing the treatment.

Another aspect of the present disclosure is directed to a control system for a dialysis machine. The control system comprises an input device for receiving information, a non-volatile memory device responsive to the input device for storing patient specific information, and a processor responsive to the memory device. The memory device carries instructions which, when executed, cause the control system to execute a method comprising receiving patient specific data at the input device, storing the received patient specific data in the non-volatile memory device, and implementing a treatment according to one of an acute care mode or a home care mode. In the case of the acute care mode of treatment, the patient specific data is deleted after implementing the treatment. In the case of the home care mode of treatment, the patient specific data is retained after implementing the treatment.

The difference between the home and acute modes of operation is the level of control of the dialysis machine that is allowed. In the clinical setting, a doctor or clinician needs flexibility in adjusting the machine, but in the home a user's control is restricted to reduce the potential for self-inflicted harm. Physicians and clinicians, for example, need to be able to modify prescription information and adjust machine settings in areas that could cause harm to a patient. The home use mode, on the other hand, restricts the ability of a user to modify prescription and machine settings that could harm the patient. Additionally, privacy concerns in a clinical setting may mandate that a patient's personal information stored in memory should be deleted as soon as that user has ceased using the machine. This information should also be deleted to prevent a situation where the prescription data is not updated for a new patient. In a home setting, however, such personal information can be retained because the risk of disclosure is minimized. Retaining such information in the home use setting also minimizes manual entry mistakes.

Examples of patient information that are entered include patient name and/or ID number, access type (single or dual lumen), blood flow rate, treatment duration, sorb cartridge type, dialysate flow rate, dialysate temperature, dialysate concentration, dialyzer information, patient pre and goal weight and/or ultra-filtration rate, heparin bolus and/or flow rate, and dialysate chemical prescription. Some parameters may be adjustable only at startup while others may be adjustable during dialysis. Examples of parameters that might be adjustable during treatment include duration, ultra-filtration goal, blood flow rate, heparin flow rate, heparin delivery end time, dialysate temperature set point, and dialysate flow rate. Again, some parameters that a physician or clinician will be permitted to adjust will not be available to the home user or the ranges over which parameters can be adjusted will be limited for the home user.

Parameters that a home user cannot access, such as prescription parameters, can be adjusted by trained personnel in the home or a diskette or other memory device with prescription information could be provided to the user by the doctor in order to change the prescription information.

### DESCRIPTION OF THE FIGURES

For the invention to be easily understood and readily practiced, the invention will now be described, for purposes of illustration and not limitation, in conjunction with the following figures wherein:

FIG. 1 is a block diagram of a dialysis system according to the teachings of the present invention; and

FIGs. 2 - 6 are screen shots of how a user may interact with the dialysis machine illustrated in FIG. 1.

### DESCRIPTION OF A PREFERRED EMBODIMENT

The dialysis system shown schematically in the drawings has parts that are examples of the elements recited in the apparatus claims, and can be operated in steps that are examples of the elements recited in the method claims. The illustrated system thus includes examples of how a person of ordinary skill in the art can make and use the claimed invention. They are described here to meet the enablement and best mode requirements of the patent statute without imposing limitations that are not recited in the claims.

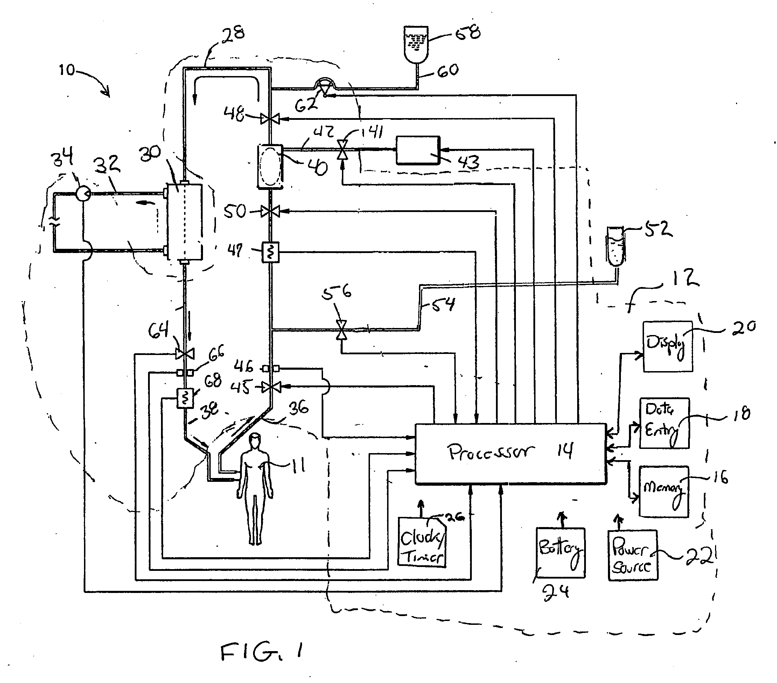

The invention encompasses a method of allowing a dialysis machine of the type shown in FIG. 1 to be set up for either a home setting or an acute setting such as a hospital or dialysis clinic.

FIG. 1 is a schematic of a dialysis system 10 of the present invention. Referring to FIG. 1, the system 10 is a renal dialysis system for the extracorporeal treatment of blood from a patient 11 whose kidney function is impaired. The illustrated embodiment of the dialysis system 10 of the present invention comprises a dialysis machine 12 as is generally known in the medical arts, and shown generally within the dotted line, plus various consumables as is known in the art.

In accordance with the present invention, the dialysis machine 12 is provided with a non-volatile memory component 16 adaptively coupled to electronic control means 14, which may be a processor. Non-volatile memory component 16 can be any form of memory component that retains stored values when external power is turned off. For example, such non-volatile memory components can be selected from the group consisting of a hard disk, flash memory, battery-backed-up RAM, or other data storage device.

Dialysis machine 12 further includes a data entry device 18, such as a keyboard, touch-screen monitor, computer mouse, or the like. Dialysis machine 12 further includes a display device 20, such as a read-out monitor, for displays of operating values of the various individual components of the dialysis machine 12. The system 10 can be provided with a power source 22, a battery back-up 24, and a clock/timer 26. The processor 14, memory 16, data entry device 18, and clock/timer 26 represent one configuration of a control system.

The dialysis system 10 comprises a blood circuit 28 through which the patient's blood travels, a dialyzer 30 that serves to separate the wastes from the blood, and a dialysate circuit 32 through which treatment fluid, specifically dialysate, travels carrying the waste away.

The dialysate circuit 32 includes a dialysate pump 34 for driving dialysate fluid through a tube set and through the dialyzer 30. The dialysate circuit 32 may further include other components such as those described in U.S. Patent Application Serial No. 11/148,928, entitled Dialysis System and filed on June 9, 2005, which is hereby incorporated by reference in its entirety.

The blood circuit 28 includes another tube set including an arterial line 36 for withdrawing blood from the patient 11 and delivering it to the dialyzer 30, and a venous line 38 for returning the treated blood to the patient 11. A blood pump 40 drives the blood around the blood circuit 28. A valve 41 is situated on a gas line 42 for supplying negative and positive pressure from a source 43 to the pump 40. The arterial line 36 also incorporates a valve 45 that can stop the flow of blood from the patient 11, an air detector 46 that can detect air in the arterial line 36, and a flow sensor 47 that measures the flow of blood. The arterial line 36 further includes a valve 48 upstream of the pump 40 and a valve 50 downstream on the pump 40. The blood pump 40 may be configured as described in U.S. Patent Application Serial No. 10/399,128, entitled Device and Methods for Body Fluid Flow Control In Extracorporeal Fluid Treatments, filed on July 28, 2003.

Other components which interact with the blood circuit 28 include a source of fluid, such as a saline bag 52, which communicates with the arterial line 36 via a branch line 54 and a valve 56 responsive to processor 14. Additionally, an anticoagulant solution such as a heparin supply 58 may communicate with the arterial line 36 through a branch line 60 and a pump 62 responsive to processor 14. It is understood by persons skilled in the art that additional elements may be added to the blood circuit 36, such as air detectors in the branch lines 54 or 60. These additional elements are omitted from the drawings for clarity of illustration. Finally, the venous line 38, which delivers the treated blood from the dialyzer 30 to the patient 11, also includes a valve 64, an air detector 66, and a flow sensor 68.

The processor 14 coordinates the operation of the dialysis system 10 by controlling the blood flow in the blood circuit 28, the dialysate flow in the dialysate circuit 32, and the flow of saline 52 or heparin 58 to the arterial line 36 via the branch lines 54 and 60, respectively. To achieve this, the processor 14 utilizes hardware and/or software configured for operation of these components and may comprise any suitable programmable logic controller or other control device, or combination of control devices, that is programmed or otherwise configured to perform as is known in the art. Thus, blood flow in the blood circuit 28 is controlled by operating the blood pump 40 and controlling the valves in the arterial and 36 and venous 38 lines. Dialysate flow in the dialysate circuit 32 is controlled by operating the dialysate pump 34.

The processor 14 is also responsive to various input signals it receives, such as input signals from one or more flow sensors 47, 68, air detectors 46, 66, and the clock/timer 26. Additionally, the processor 14 displays system status and various other treatment parameters, known in the art, on the display 20. That allows the operator to interact with the processor 14 via the data entry device 18 (which could include a touch sensitive display 20).

The present invention can be used with a variety of different commercially available dialysis machines; one such machine particularly suited for use with the present invention is a dialysis machine sold under the registered trademark ALLIENT by Renal Solutions, Inc., the assignee of the invention herein.

The dialysis system 10 must be set up before it can be used to provide dialysis treatment for a patient. The system 10 has the capability to be set up and used in either a home or a clinical care setting. When set up in the clinical setting, the operation of the machine 12 allows more flexibility for the doctor or clinician than when set up for the home setting. In the home setting, the machine 12 restricts the ability of the user to modify prescription and machine settings that could cause harm to the patient. In the home setting, the patient and prescription data is stored and is persistent so that the user does not need to reenter the values for each treatment. When set up for acute setting, the machine does not store patient and prescription data, i.e., patient specific data is not persistent. The patient specific data may be deleted automatically, with or without some type of output to the user, or may require some type of user input to confirm deletion. The deletion of the patient specific data protects patient privacy and eliminates the incidence of prescription data from the previous treatment accidentally being used for the next patient.

When the machine is powered up the first time, it comes up to the Main Menu with two button choices; "Begin Acute Treatment Setup" or "System Menu." To run in acute venue mode, the user selects "Begin Acute Treatment Setup."

The user then interacts with the machine 10 to enter a set of patient specific data which may include some or all of the patient name, ID, single or dual lumen access, blood flow rate, treatment duration, sorb cartridge type, dialysate flow rate, dialysate temperature, dialyzer information, patient pre and goal weight or UF rate, heparin bolus and flow rate information, dialysate chemical prescription, among others.

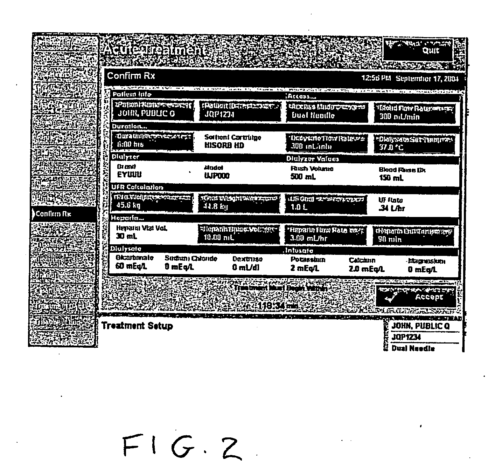

The screen shot of FIG. 2 illustrates a set of data that could be entered in one embodiment, although the present invention is not to be limited by any particular set of data or information.

Once the user begins treatment, certain parameters are available for adjustment. The user has the ability to adjust these parameters within the same predefined acceptable limits as during set up. The parameters that may be made available for adjustment during treatment include:
- Duration
- Ultra-Filtration Goal
- Blood Flow Rate
- Heparin Flow Rate
- Heparin Delivery End Time
- Dialysate Temperature Set Point
- Dialysate Flow Rate

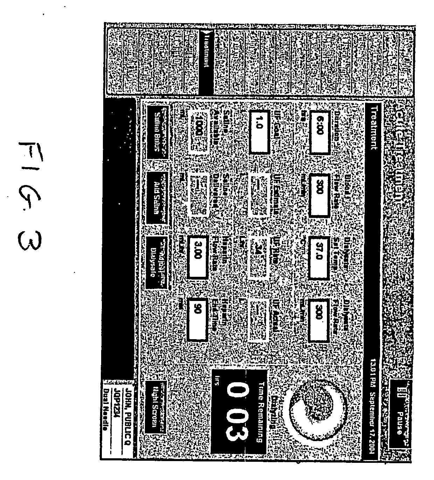

FIG. 3 is a screen shot that shows the control of those parameters as white boxes with black borders. In addition to these controls, the acute venue also allows the user to adjust the dialysate concentration during treatment.

At the end of the treatment in the acute care setting, none of the entered data is stored for the next treatment except data that the user has set up as default settings to be used for all treatments. Access to the default parameters is controlled through a menu whose access is restricted by a password.

In contrast to the acute venue mode, the home venue restricts the user's ability to make changes to the prescription data entered. To enter prescription data for home treatment, at the main menu, the user selects "System Menu" and then "Restricted Menu". This menu is restricted by password.

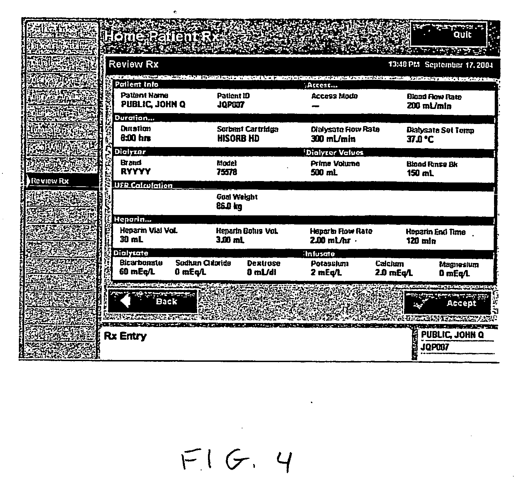

After entering the appropriate password, the user may choose to change to "Home Treatment Mode". The user then interacts with the machine to enter data such as the patient name, ID, single lumen access, blood flow rate, treatment duration, sorb cartridge type, dialysate flow rate, dialysate temperature, dialyzer information, patient pre and goal weight or UF rate, heparin bolus and flow rate information, and dialysate chemical prescription the same as in acute treatment mode. The reader will recognize that this data is a subset of the first set of data discussed above. The exceptions are the access mode where only single lumen is allowed, so the blood flow rate range is not as wide; and the other exception is the UF goal and rate entry. In home treatment mode, only the patient goal weight is entered, not the pre-weight or UF goal. The screen shot of FIG. 4 shows these values after they have been entered:

When the prescription parameters have been reviewed by the user and accepted, the machine stores the values to be used during each treatment. The user returns to the main screen and can then begin a home treatment.

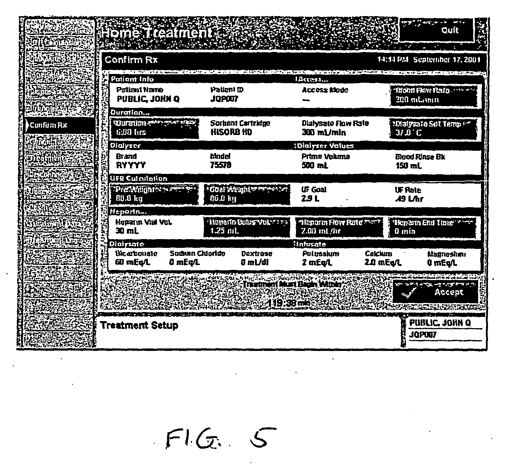

When a Home Treatment is selected, the user is given the opportunity to modify some of the prescription parameters within a limited range prior to treatment without password protection. The parameters that they may adjust and the adjustment limits are, for example:
◆ Patient Pre-Weight Limits: Goal Weight to Goal Weight + 10%
◆ Patient Goal Weight Limit: must be less than Patient Pre-weight but not less than Pre-weight - 10%
◆ Duration Limit: Upper limit dictated by cartridge used. May not reduce below prescription value.
◆ Blood Flow Rate Limit: 150ml/min to 200ml/min. User may only decrease from prescription value.
◆ Heparin flow rate Limit: 0-3 ml/Hr. User can only decrease the rate, not increase it.
◆ Dialysate temperature set point Limit: 34°C to 39°C
◆ Heparin bolus volume Limit: 0 - 10ml. User can only adjust down.
◆ Heparin end time Limit: 0 to 120 Mins. User can only adjust up to 120 Mins.
The screen shot of FIG. 5 shows those parameters that may be modified as dark buttons.
Once treatment has begun, the user can only modify the following parameters:
- Duration May be extended up to one hour. The user may not reduce it lower than the prescription setting
- Blood Flow Rate May only be reduced during treatment
- Heparin Flow Rate May only be reduced during treatment
- Heparin Delivery End Time May only adjust up to 120 Mins
- Dialysate Temperature Set Point Limit 34°C to 39°C

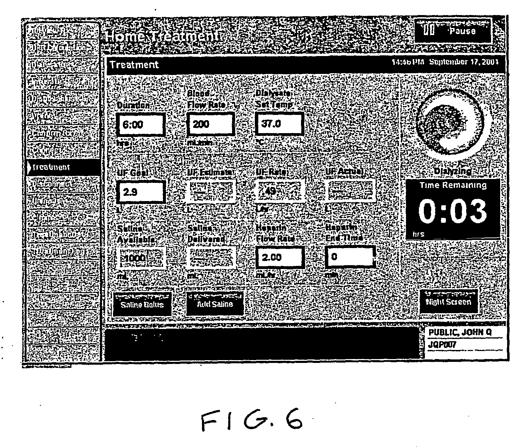

The screen shot of FIG. 6 shows these controls as white boxes with black borders. In addition to these controls the user may select saline bolus, but dialysate concentration adjustment is not an option.

At the start of each treatment, the patient and prescription values are reloaded. Any adjustments made prior to the beginning of treatment or made during treatment are not reloaded.

Home dialysis treatments require a greater level of safety features than dialysis treatment in the presence of a trained and skilled practitioner. The home user must be protected from making adjustments to the machine and prescription parameters that could cause harm to themselves.

At the same time, skilled practitioners in a hospital or dialysis clinic demand a machine with greater flexibility to treat patients with widely varying needs. There are also patient privacy concerns that must be protected in different ways for each of these venues.

This invention provides one machine that may be set up for either the acute venue or the home venue. The data that is stored, the patient identification data and the amount of flexibility that the user has available are different for the two venues.

Acute venue provides the user with maximum flexibility, without storing patient specific data, while the home venue reduces the likelihood that the home user will inadvertently harm themselves.

By pre-populating the home user prescription data, there is less chance that the home user may enter incorrect values. The limits also help protect the user from adjusting parameters outside of a safe range.

Updating the home patient parameters may be done onsite by trained personnel. Alternatively, a diskette or other memory device like a memory stick may be inserted into the machine. At power up, the machine can read the memory device and load new patient prescription parameters to be used for new treatments if the patient name matches and the new prescription data is newer than the existing prescription data.

The foregoing description of a preferred embodiment of the invention is presented by way of illustration and not by way of limitation. It is to be understood that any changes, modifications, and equivalents that come within the invention are to be covered by the following claims.

## Claims

1. A dialysis machine (12) comprising a first pump (40) and a first plurality of valves for moving blood through a blood circuit (28); a second pump (34) for moving dialysate through a dialysate circuit (32); said dialysis machine **characterized by** a control system comprising an input device (18) for receiving information; a non- volatile memory device (16) responsive to said input device (18) for storing patient specific information; and a processor (14) responsive to said memory device (16), said control system configured to:
receive patient specific data;
store said received patient specific data;
send instructions to said first and second pumps (40, 34) and said first plurality of valves to implement a treatment according to one of an acute care mode or a home care mode; and
delete said patient specific data after implementing said acute care mode of treatment and retain said patient specific data after implementing said home care mode of treatment.

2. The machine (12) of claim 1 wherein said receiving patient specific data comprises enabling the reception of a first set of patient specific data when in the acute care mode, and enabling the reception of a subset of said first set of patient specific data when in the home care mode.

3. The machine (12) of claim 2 wherein said enabling the reception comprises enabling data entry at certain portions of a display (20) while disabling data entry at certain other portions of said display (20).

4. The machine (12) of claim 1 wherein said receiving patient specific data comprises enabling the reception of a first range for patient specific parameters when in the acute care mode, and enabling the reception of a subset of said first range for patient specific parameters when in the home care mode.

5. The machine (12) of claim 1 additionally comprising receiving a modification to a patient specific parameter within said patient specific data during implementation of a treatment.

6. The machine (12) of claim 5 wherein said receiving a modification to a patient specific parameter comprises enabling the reception of a first range for patient specific parameters when in the acute care mode, and enabling the reception of a subset of said first range for patient specific parameters when in the home care mode.

## Patentansprüche

1. Eine Dialysemaschine (12) umfassend eine erste Pumpe (40) und eine erste Vielzal von Ventilen für das Bewegen von Blut durch einen Blutkreislauf (28); eine zweite Pumpe (34) für das Bewegen eines Dialysats durch einen Dialysatkreislauf (32); die Dialysatmaschine **gekennzeichnet durch** ein Kontrollsystem umfassend ein Eingabegerät (18) zum Empfangen von Informationen; ein permamentes Speichermedium (16) zum Speichern von Patienten-spezifischen Informationen, welches von dem Eingabegerät (18) ansprechbar ist; und ein Prozessor (14), welcher von dem Speichermedium (16) ansprechbar ist, wobei das Kontrollsystem ausgelegt ist für:
empfangen von Patienten-spezifischen Daten;
speichern der empfangenen Patienten-spezifischen Daten;
senden von Instruktionen zu der ersten und zweiten Pumpe (40, 34) und zu der ersten Vielzahl von Ventilen um eine Behandlung entsprechend einem akuten Behandlungsmodus oder einem Heim-Behandlungsmodus zu implementieren; und
löschen der Patienten-spezifischen Daten nach der Implementierung des akuten Behandlungsmodus und weiterhin speichern der Patienten-spezifischen Daten nach der Implementierung des Heim-Behandlungsmodus.

2. Die Maschine (12) des Anspruch 1, wobei das Empfangen der Patienten-spezifischen Daten, falls in dem akuten Behandlungsmodus, aktivieren des Empfangs eines ersten Sets von Patienten-spezifischen Daten umfasst, und, falls in dem Heim-Behandlungsmodus, aktivieren des Empfangs einer Untermenge des ersten Sets von Patienten-spezifischen Daten umfasst.

3. Die Maschine (12) des Anspruch 2, wobei das Aktivieren des Empfangs ein Aktivieren einer Dateneingabe an bestimmten Teilen einer Bildschirmanzeige (20) während eines Inaktivieren einer Dateneingabe an anderen Teilen der Bildschirmanzeige (20) umfasst.

4. Die Maschine (12) des Anspruch 1, wobei das Empfangen von Patienten-spezifischen Daten, falls in dem akuten Behandlungsmodus, aktivieren des Empfangs eines ersten Bereichs für Patienten-spezifische Parameter umfasst, und, falls in dem Heim-Behandlungsmodus, aktivieren des Empfangs einer Untermenge des ersten Bereichs für Patienten-spezifische Parameter umfasst.

5. Die Maschine (12) des Anspruch 1, zusätzlich umfassend:
empfangen einer Modifikation eines Patienten-spezifische Parameters innerhalb der Patienten-spezifischen Daten während einer Implementierung einer Behandlung.

6. Die Maschine (12) des Anspruch 5, wobei das Empfangen einer Modifikation eines Patienten-spezifische Parameters, falls in dem akuten Behandlungsmodus, aktivieren des Empfangs eines ersten Bereichs für Patienten-spezifische Parameter umfasst, und, falls in dem Heim-Behandlungsmodus, aktivieren des Empfangs einer Untermenge des ersten Bereichs für Patienten-spezifische Parameter umfasst.

## Revendications

1. Appareil de dialyse (12) comprenant une première pompe (40) et une première pluralité de valves pour faire circuler du sang à travers un circuit sanguin (28) ; une seconde pompe (34) pour faire circuler un dialyse à travers un circuit de dialyse (32) ; ledit appareil de dialyse étant **caractérisé par** un système de commande comprenant un dispositif d'entrée (18) pour recevoir des informations ; un dispositif de mémoire non volatile (16) réagissant audit dispositif d'entrée (18) pour mémoriser des informations spécifiques au patient ; et un processeur (14) réagissant audit dispositif de mémoire (16), ledit système de commande étant configuré pour :
recevoir des données spécifiques au patient ;
mémoriser lesdites données spécifiques au patient reçues ;
envoyer des instructions auxdites première et seconde pompes (40, 34) et à ladite première pluralité de valves pour mettre en oeuvre un traitement en fonction d'un mode de soins aigus ou d'un mode de soins à domicile ; et
effacer lesdites données spécifiques au patient après la mise en oeuvre dudit mode de traitement de soins aigus et conserver les données spécifiques au patient après la mise en oeuvre dudit mode de traitement de soins à domicile.

2. Appareil (12) selon la revendication 1, dans lequel la réception desdites données spécifiques au patient comprend l'activation de la réception d'un premier ensemble de données spécifiques au patient en mode de soins aigus et l'activation de la réception d'un sous-ensemble dudit premier ensemble de données spécifiques au patient en mode de soins à domicile.

3. Appareil (12) selon la revendication 2, dans lequel ladite activation de la réception comprend l'activation de la saisie de données dans certaines portions d'un affichage (20) tout en désactivant la saisie de données dans certaines autres portions dudit affichage (20).

4. Appareil (12) selon la revendication 1, dans lequel ladite réception de données spécifiques au patient comprend l'activation de la réception d'une première plage de paramètres spécifiques au patient en mode de soins aigus, et l'activation de la réception d'un sous-ensemble de ladite première plage de paramètres spécifiques au patient dans le mode de soins à domicile.

5. Appareil (12) selon la revendication 1, comprenant en outre la réception d'une modification d'un paramètre spécifique au patient dans lesdites données spécifiques au patient au cours de la mise en oeuvre d'un traitement.

6. Appareil (12) selon la revendication 5, dans lequel ladite réception d'une modification d'un paramètre spécifique au patient comprend l'activation de la réception d'une première plage de paramètres spécifiques au patient en mode de soins aigus, et l'activation de la réception d'un sous-ensemble de ladite première plage de paramètres spécifiques au patient en mode de soins à domicile.
